# EUROPEAN PATENT SPECIFICATION

(11) **EP 1 899 331 B1**
(45) Date of publication and mention of the grant of the patent: **25.11.2009**
(21) Application number: 06779767.0
(22) Date of filing: 20.06.2006
(51) Int. Cl.: C07D 413/10, C07D 413/14, A61P 31/04, A61K 31/4178

(54) **HOMOMORPHOLINE OXAZOLIDINONES AS ANTIBACTERIAL AGENTS**
HOMOMORPHOLINOXAZOLIDINONE ALS ANTIBAKTERIELLE MITTEL
OXAZOLIDINONES D'HOMOMORPHOLINE EN TANT QU'AGENTS ANTIBACTERIENS

(30) Priority: 29.06.2005 US 694862 P
(43) Date of publication of application: 19.03.2008
(73) Proprietor: Pharmacia & Upjohn Company LLC, Kalamazoo, MI 49001-0199 (US)
(72) Inventor: JOSYULA, Vara Prasad Venkata Nagendra, Ann Arbor, MI 48105 (US); BOYER, Frederick Earl Jr., Ann Arbor, MI 48105 (US); KIM, Ji-Young, Ann Arbor, MI 48105 (US)
(74) Representative: Kingsbury, Oliver William
(86) International application number: PCT/IB2006/001730
(87) International publication number: WO 2007/000644

(56) References cited:
- WO-A-00/32599
- WO-A-01/46185
- WO-A-95/07271
- WO-A-96/15130
- WO-A-03/072575
- WO-A2-02/02095
- WO-A2-03/093247

## Description

### FIELD OF INVENTION

The present invention relates to a new class of oxazolidinone derivatives, to these compounds for use as antibacterial agents, to pharmaceutical compositions containing these compounds and to methods for their preparation.

### BACKGROUND OF THE INVENTION

Antibacterial resistance is a global clinical and public health problem that has emerged with alarming rapidity in recent years and undoubtedly will increase in the near future. Resistance is a problem in the community as well as in health care settings, where transmission of bacteria is greatly amplified. Because multiple drug resistance is a growing problem, physicians are now confronted with infections for which there is no effective therapy. As result, structurally novel antibacterials with a new mode of action have become increasingly important in the treatment of bacterial infections.

Among newer antibacterial agents, oxazolidinone compounds are the most recent synthetic class of antimicrobials. This invention provides a new class of oxazolidinone derivatives containing a homomorphline ring, which are active against a number of human and veterinary pathogens, including multiple resistant strains of bacteria.

### INFORMATION DISCLOSURE

WO 9323384, WO 9507271, WO 20028084, WO 2003072553, WO 2003072576, WO 2003072575, WO 200142229, WO 200264575, WO 9615130, WO 200216960, WO 200027830, WO 200146185, WO 200281469, WO 200281470, WO 2001080841, WO 2003084534, WO 2003093247, WO 200202095, WO 200230395, WO 200272066, WO 2003063862, WO 2003072141, WO 2003072081, WO 2003119817, WO 2003008389, WO 2003007870, WO 200206278, WO 200032599, WO 9924428, WO 2004014392, WO 2004002967, WO 2004009587, WO 2004018439, US Patent Application Publication No. US 2004/0044052, US Patent No. 5547950, US Patent No. 5700799, DE 10034627 disclose oxazolidinone compounds having antibacterial activity useful for treating microbial infections.

### SUMMARY OF THE INVENTION

The present invention provides a compound of formula I 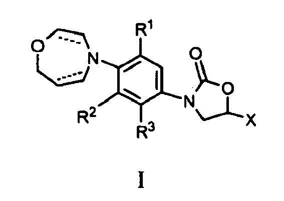 or a pharmaceutically acceptable salt thereof wherein:
X is -C(=O)_{NHR}⁴_{;}
R¹, R², R³ are independently H or F;
R⁴ is H, C₁₋₆ alkyl, or OC₁₋₆alkyl;
each dotted line "..." is independently an optional single bond; and at each occurrence, C₁₋₆alkyl is optionally substituted by 1-3 halo, OH, OC₁₋₄akyl, CN, N₃, O(C=O)Cm alkyl, NH₂, NHC(=O)C₁₋₄ alkyl, or C(=O)C₁₋₄ alkyl.

In other aspects, the present invention also provides:
a pharmaceutical composition which comprises a pharmaceutically acceptable carrier and a compound of formula I,
a compound of formula I or a pharmaceutically acceptable salt thereof for treating microbial infections in a mammal, and
a use of a compound of formula I or a pharmaceutically acceptable salt thereof to prepare a medicament for treating microbial infections.

The invention may also provide novel intermediates and novel processes that are useful for preparing compounds of formula I.

### DETAILED DESCRIPTION OF THE INVENTION

Unless otherwise stated, the following terms used in the specification and claims have the meanings given below:

The carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, C₁₋₆ alkyl refers to alkyl of one to six carbon atoms, inclusive.

The term alkyl, or alkenyl, etc. refer to both straight and branched groups, but reference to an individual radical such as "propyl" embraces only the straight chain radical, a branched chain isomer such as "isopropyl" being specifically referred to.

The term "halo" refers to fluoro (F), chloro (Cl), bromo (Br), or iodo (I).

The term "a pharmaceutically acceptable salt" of a compound means a salt that is pharmaceutically acceptable and that possesses the desired pharmacological activity of the parent compound.

The term "pharmaceutically acceptable carrier" means a carrier that is useful in preparing a pharmaceutical composition that is generally safe, non-toxic and neither biologically nor otherwise undesirable, and includes a carrier that is acceptable for veterinary use as well as human pharmaceutical use. "A pharmaceutically acceptable carrier" as used in the specification and claims includes both one and more than one such carrier.

The term "mammal" refers to human or warm-blooded animals including livestock and companion animals. Livestock refers to animals suitable for human meat consumption. Examples include pigs, cattle, chickens, fish, turkeys, rabbits, etc. Companion animals refer to animals kept as pets such as dogs, cats, etc.

The term "optional" or "optionally" means that the subsequently described event or circumstance may, but need not, occur, and that the description includes instances where the event or circumstance occurs and instances in which it does not.

The term "treating" or "treatment" of a disease includes: (1) preventing the disease, i.e. causing the clinical symptoms of the disease not to develop in a mammal that may be exposed to or predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

The term "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "leaving group" has the meaning conventionally associated with it in synthetic organic chemistry i.e., an atom or group capable of being displaced by a nucleophile and includes halogen, alkylsulfonyloxy, ester, or amino such as chloro, bromo, iodo, mesyloxy, tosyloxy, trifluorosulfonyloxy, methoxy, N,O-dimethylhydroxyl-amino, and the like.

Compounds that have the same molecular formula but differ in the nature or sequence of bonding of their atoms or the arrangement of their atoms in space are termed "isomers". Isomers that differ in the arrangement of their atoms in space are termed "stereoisomers".

It will be appreciated by those skilled in the art that compounds of the invention having a chiral center may exist in and be isolated in optically active and racemic forms. Some compounds may exhibit polymorphism. It is to be understood that the present invention encompasses any racemic, optically-active, polymorphic, tautomeric, or stereoisomeric form, or mixture thereof, of a compound of the invention, which possesses the useful properties described herein, it being well known in the art how to prepare optically active forms (for example, by resolution of the racemic form by recrystallization techniques, by synthesis from optically-active starting materials, by chiral synthesis, or by chromatographic separation using a chiral stationary phase) and how to determine antiviral activity using the standard tests described herein, or using other similar tests which are well known in the art.

The compounds of the present invention are generally named according to the IUPAC or CAS nomenclature system.

Abbreviations which are well known to one of ordinary skill in the art may be used (e.g. "Ph" for phenyl, "Me" for methyl, "Et" for ethyl, "h" for an hour or hours and "rt" for room temperature).

Specific and preferred values listed below for radicals, substituents, and ranges, are for illustration only; they do not exclude other defined values or other values within defined ranges for the radicals and substituents.

Specifically, alkyl denotes both straight and branched groups; but reference to an individual radical such as "propyl" embraces only the straight chain radical, a branched chain isomer such as "isopropyl" being specifically referred to.

Specifically, alkyl is methyl, ethyl, propyl, isopropyl, butyl, iso-butyl, sec-butyl, and their isomeric forms thereof.

Specifically, halo is fluoro (F), or chloro (Cl).

Specifically, R¹, R² and R³ are independently H or F.

Specifically, R⁴ is H, -CH₃, or -CH₂CH₃.

Specifically, R⁴ is -OCH₃, or -OCH₂CH₃.

Specifically the present invention provides a compound of formula Ia

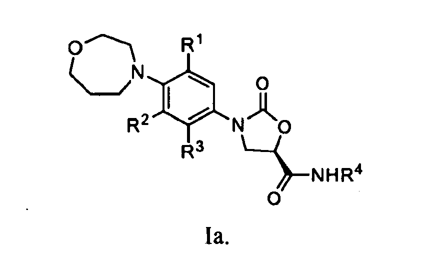

Scheme I describes the preparation of compounds of the present invention. The starting materials are prepared by procedures described in these schemes or by procedures known to one of ordinary skill in the art.

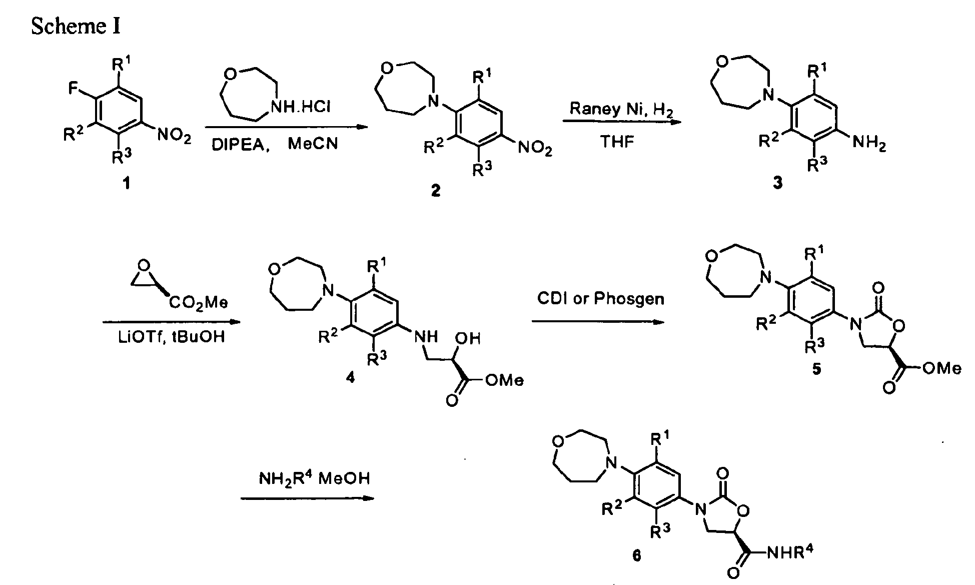

According to Scheme I, the appropriately substituted 4-fluoronitrobenzene **(1)** is converted to the corresponding anilines **(2)** via aromatic nucleophilic displacement by treating with the corresponding amine in the presence of a base. The nitro compound **(2)** is reduced to its corresponding amino compound **(3)** via chemical (using metal like Fe and ammonium chloride) or catalytic reduction (catalyst like Raney nickel in the presence of hydrogen). Amine **(3)** is reacted with an appropriately protected (R)-methyl glycidate and a Lewis acid such as lithium triflate as described in US Patent Application Publication No. US2004/0044052. The amino alcohol **(4)** can then be ring closed to give aryl oxazolidinone **(5)** using methods known to one skilled in the art. For instance, treatment of structure 4 with 1,1'-carbonyldiimidazole in solvent such as acetonitrile or tetrahydrofuran at an appropriate temperature, typically in the range of 20 °C to 60 °C or with phosgene in a solvent such as toluene or methylene chloride, or mixture thereof, in the presence of a base such as triethylamine at an appropriate temperature, typically in a range from -10 °C to 25 °C affords the oxazolidinone **(5).** Subsequent treatment of oxazolidinone ester **(5)** with ammonia or optionally substituted amines (RNH₂) in a suitable solvent such as methanol or acetonitrile affords amides **(6).** Similarly, treatment of ester (5) with O-alkylhydroxylamines or hydrazines gives the hydroxamate (R = O-alkyl) or hydrazide (R = NH₂) respectively.

### Medical and Veterinary Uses

It is known that as a chemical compound class, oxazolidinones generically inhibit monoamine oxidase (MAO), the enzyme responsible for preventing acute blood pressure elevation by the endogenous and dietary amine, tyramine. Accordingly, there is a demand to discover oxazolidinone antibiotics, which possess minimum MAO inhibitory activity to lower risk of potential drug-drug interactions. It has been discovered that, the compound of the present invention has unexceptedly weak MAO inhibitory activity, which indicates it possess the capacity to minimize or eliminate potential drug-drug interactions since strong inhibition of monoamine oxidase can result in altered clearance rates for other compounds normally metabolized by it, including several pharmaceuticals.

The compound of the present invention may be used for the treatment of infectious, Gram-positive bacterial infections caused by a variety of bacterial organisms, including those that require long-term therapy (>28 days).

Examples of the bacterial organisms include gram-positive bacteria such as multiple resistant staphylococci, for example *S. aureus and S. epidermidis*; multiple resistant streptococci, for example *S. pneumoniae* and *S. pyogenes*; and multiple resistant Enterococci, for example *E. faecalis*; gram negative aerobic bacteria such as Haemophilus, for example *H. influenzae* and Moraxella, for example *M. catarrhalis*; as well as anaerobic organisms such as bacteroides and clostridia species, and acid-fast organisms such as Mycobacteria, for example *M. tuberculosis*; and/or *Mycobacterium avium*. Other examples include Escherichia, for example *E. coli*. intercellular microbes, for example Chlamydia and Rickettsiae.

Examples of infections that may be treated with the compound of the present invention include central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynecological infections, septicemia, bone and joint infections, skin and skin structure infections, bacterial endocarditis, bums, antibacterial prophylaxis of surgery, and antibacterial prophylaxis in immunosuppressed patients, such as patients receiving cancer chemotherapy, or organ transplant patients. Specifically, infectious diseases that may be treated with the compound of the present invention are gram-positive infections such as osteomyelitis, endocarditis and diabetic foot.

### Antibacterial activity

The *in vitro* antibacterial activity of the compounds of the present invention may be assessed by following procedures recommended in (1) National Committee for Clinical Laboratory Standards (Jan. 2003), *Methods for dilution antimicrobial tests for bacteria that grow aerobically*, Approved Standard (6^{th} ed), M7-A6, NCCLS, Wayne, PA; (2) National Committee for Clinical Laboratory Standards (Mar. 2001), *Methods for antimicrobial susceptibility testing of anaerobic bacteria*, Approved Standard (5^{th} ed), M11-A4, NCCLS, Wayne, PA; (3) National Committee for Clinical Laboratory Standards (Jan.2003), *MIC testing supplemental tables*, M100-S13 (for use with M7-A6), NCCLS, Wayne, PA; and (4) Murray PR, Baron EJ, Jorgensen JH, et al. Manual of Clinical Microbiology (8th ed) Washington, DC: American Society for Microbiology Press, 2003. The antibacterial activity can be presented in the form of MIC value. The MIC value is the lowest concentration of drug, which prevented macroscopically visible growth under the conditions of the test.

**Table 1**

| (Minimum Inhibitory concentrations µg/mL) | | | |
|---|---|---|---|
| Example | *S. aureus* | *S. pyogenes* | *S. pneumoniae* |
| | (UC-76) | (C203) | (SV-1) |
| 1 | 8 | 8 | 16 |
| 5 | 4 | 4 | 8 |
| 6 | 4 | 4 | 8 |

### Pharmaceutical Salts

The compound of formula I may be used in its native form or as a salt. In cases where forming a stable nontoxic acid or base salt is desired, administration of the compound as a pharmaceutically acceptable salt may be appropriate. Examples of pharmaceutically acceptable salts of the present invention include inorganic salts such as hydrochloride, hydrobromide, sulfate, nitrate, bicarbonate, carbonate salts, and organic salts such as tosylate, methanesulfonate, acetate, citrate, malonate, tartarate, succinate, benzoate, ascorbate, etoglutarate, and glycerophosphate.

Pharmaceutically acceptable salts may be obtained using standard procedures well known in the art, for example, reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

### Routes of Administration

In therapeutic use for treating, or combating, bacterial infections in a mammal (i.e. human and animals), a compound of the present invention or its pharmaceutical compositions can be administered orally, parenterally, topically, rectally, transmucosally, or intestinally.

Parenteral administrations include indirect injections to generate a systemic effect or direct injections to the afflicted area. Examples of parenteral administrations are subcutaneous, intravenous, intramuscular, intradermal, intrathecal, intraocular, intranasal, intravetricular injections or infusions techniques.

Topical administrations include the treatment of infectious areas or organs readily accessibly by local application, such as, for example, eyes, ears including external and middle ear infections, vaginal, open wound, skins including the surface skin and the underneath dermal structures, or other lower intestinal tract. It also includes transdermal delivery to generate a systemic effect.

The rectal administration includes the form of suppositories.

The transmucosal administration includes nasal aerosol or inhalation applications.

The preferred routes of administration are oral and parenteral.

### Composition/Formulation

Pharmaceutical compositions of the present invention may be manufactured by processes well known in the art, *e*.*g*., by means of conventional mixing, dissolving, granulation, dragee-making, levigating, emulsifying, encapsulating, entrapping, lyophilizing processes or spray drying.

Pharmaceutical compositions for use in accordance with the present invention may be formulated in conventional manner using one or more physiologically acceptable carriers comprising excipients and auxiliaries, which facilitate processing of the active compound into preparations, which can be used pharmaceutically. Proper formulation is dependent upon the route of administration chosen.

For oral administration, the compound can be formulated by combining the active compound with pharmaceutically acceptable carriers well known in the art. Such carriers enable the compound of the invention to be formulated as tablets, pills, lozenges, dragees, capsules, liquids, solutions, emulsions, gels, syrups, slurries, suspensions and the like, for oral ingestion by a patient. A carrier can be at least one substance which may also function as a diluent, flavoring agent, solubilizer, lubricant, suspending agent, binder, tablet disintegrating agent, and encapsulating agent. Examples of such carriers or excipients include, but are not limited to, magnesium carbonate, magnesium stearate, talc, sugar, lactose, sucrose, pectin, dextrin, mannitol, sorbitol, starches, gelatin, cellulosic materials, low melting wax, cocoa butter or powder, polymers such as polyethylene glycols and other pharmaceutical acceptable materials.

Dragee cores are provided with suitable coatings. For this purpose, concentrated sugar solutions may be used which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical compositions, which can be used orally, include push-fit capsules made of gelatin, as well as soft, sealed capsules made of gelatin and a plasticizer, such as glycerol or sorbitol. The push-fit capsules can contain the active ingredients in admixture with a filler such as lactose, a binder such as starch, and/or a lubricant such as talc or magnesium stearate and, optionally, stabilizers. In soft capsules, the active compound may be dissolved or suspended in suitable liquids, such as fatty oils, liquid paraffin, liquid polyethylene glycols, cremophor, capmul, medium or long chain mono-, di- or triglycerides. Stabilizers may be added in these formulations, also.

Liquid form compositions include solutions, suspensions and emulsions. For example, there may be provided solutions of the compound of this invention dissolved in water and water-propylene glycol and water-polyethylene glycol systems, optionally containing suitable conventional coloring agents, flavoring agents, stabilizers and thickening agents.

The compound may also be formulated for parenteral administration, *e*.*g*., by injections, bolus injection or continuous infusion. Formulations for parenteral administration may be presented in unit dosage form, *e*.*g*., in ampoules or in multi-dose containers, with an added preservative. The compositions may take such forms as suspensions, solutions or emulsions in oily or aqueous vehicles, and may contain formulating materials such as suspending, stabilizing and/or dispersing agents.

For injection, the compound of the invention may be formulated in aqueous solution, preferably in physiologically compatible buffers or physiological saline buffer. Suitable buffering agents include trisodium orthophosphate, sodium bicarbonate, sodium citrate, N-methylglucamine, L(+)-lysine and L(+)-arginine.

Parenteral administrations also include aqueous solutions of a water soluble form, such as, without limitation, a salt, of the active compound. Additionally, suspensions of the active compound may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances, which increase the viscosity of the suspension, such as sodium carboxymethyl cellulose, sorbitol, or dextran. Optionally, the suspension may also contain suitable stabilizers and/or agents that increase the solubility of the compound to allow for the preparation of highly concentrated solutions.

Alternatively, the active ingredient may be in powder form for constitution with a suitable vehicle, *e*.*g*., sterile, pyrogen-free water, before use.
For suppository administration, the compound may also be formulated by mixing the agent with a suitable non-irritating excipient, which is solid at room temperature but liquid at rectal temperature and therefore will melt in the rectum to release the drug. Such materials include cocoa butter, beeswax and other glycerides.

For administration by inhalation, compound of the present invention can be conveniently delivered through an aerosol spray in the form of solution, dry powder, or suspensions. The aerosol may use a pressurized pack or a nebulizer and a suitable propellant. In the case of a pressurized aerosol, the dosage unit may be controlled by providing a valve to deliver a metered amount. Capsules and cartridges of, for example, gelatin for use in an inhaler may be formulated containing a power base such as lactose or starch.

For topical applications, the pharmaceutical composition may be formulated in a suitable ointment containing the active component suspended or dissolved in one or more carriers. Carriers for topical administration of the compounds of this invention include, but are not limited to, mineral oil, liquid petrolatum, white petrolatum, propylene glycol, polyoxyethylene, polyoxypropylene compound, emulsifying wax and water. Alternatively, the pharmaceutical compositions can be formulated in a suitable lotion such as suspensions, emulsion, or cream containing the active components suspended or dissolved in one or more pharmaceutically acceptable carriers. Suitable carriers include, but are not limited to, mineral oil, sorbitan monosterate, polysorbate 60, cetyl esters wax, ceteary alcohol, 2-octyldodecanol, benzyl alcohol and water.

For ophthalmic and otitis uses, the pharmaceutical compositions may be formulated as micronized suspensions in isotonic, pH adjusted sterile saline, or preferably, as solutions in isotonic, pH adjusted sterile saline, either with or without a preservative such as a benzylalkonium chloride. Alternatively, for ophthalmic uses, the pharmaceutical compositions may be formulated in an ointment such as petrolatum.

In addition to the formulations described previously, the compound may also be formulated as depot preparations. Such long acting formulations may be in the form of implants. A compound of this invention may be formulated for this route of administration with suitable polymers, hydrophobic materials, or as a sparing soluble derivative such as, without limitation, a sparingly soluble salt.

Additionally, the compound may be delivered using a sustained-release system. Various sustained-release materials have been established and are well known by those skilled in the art. Sustained-release capsules may, depending on their chemical nature, release the compound for 24 hours or for up to several days.

### Dosage

Pharmaceutical compositions suitable for use in the present invention include compositions wherein the active ingredients are contained in an amount sufficient to achieve the intended purpose, *i*.*e*., the treatment or prevent of infectious diseases. More specifically, a therapeutically effective amount means an amount of compound effective to prevent, alleviate or ameliorate symptoms of disease or prolong the survival of the subject being treated.

The quantity of active component, that is the compound of this invention, in the pharmaceutical composition and unit dosage form thereof may be varied or adjusted widely depending upon the manner of administration, the potency of the particular compound and the desired concentration. Determination of a therapeutically effective amount is well within the capability of those skilled in the art. Generally, the quantity of active component will range between 0.5% to 90% by weight of the composition.

Generally, a therapeutically effective amount of dosage of active component will be in the range of about 0.1 to about 400 mg/kg of body weight/day, preferably about 0.1 to about 100mg/kg of body weight/day, more preferably about 1.0 to about 50 mg/kg of body weight/day. It is to be understood that the dosages may vary depending upon the requirements of each subject and the severity of the bacterial infection being treated. In average, the effective amount of active component is about 200 mg to 800 mg and preferable 600 mg per day.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator or by application of a plurality of drops into the eye.

Also, it is to be understood that the initial dosage administered may be increased beyond the above upper level in order to rapidly achieve the desired plasma concentration. On the other hand, the initial dosage may be smaller than the optimum and the daily dosage may be progressively increased during the course of treatment depending on the particular situation. If desired, the daily dose may also be divided into multiple doses for administration, e.g., two to four times per day.

In cases of local administration or selective uptake, the effective local concentration of the drug may not be related to plasma concentration and other procedures know in the art may be used to determine the desired dosage amount.

The compounds of this invention can be prepared in accordance with the methods discussed below. All of the starting materials are either commercially available or can be prepared by procedures that would be well known to one of ordinary skill in organic chemistry. Also, in the discussion the preparations below, the following abbreviations have the following meanings. If an abbreviation is not defined, it has its generally accepted meaning.

| | | |
|---|---|---|
| bm | = | broad multiple |
| bd | = | broad doublet |
| bs | = | broad singlet |
| bt | = | broad triplet |
| CDI | = | 1,1 *O*-carbodiimidazole |
| d | = | doublet |
| dd | = | doublet of doublets |
| dq | = | doublet of quartets |
| dt | = | doublet of triplets |
| dm | = | doublet of multiplets |
| DMF | = | dimethylformamide |
| DMAP | = | dimethylaminopyridine |
| DIEA | = | diisopropylethylamine |
| DMSO | = | dimethyl sulfoxide |
| eq. | = | equivalents |
| g | = | grams |
| h | = | hour or hours |
| HPLC | = | high pressure liquid chromatography |
| HATU | = | N-[(dimethylamino)-1H-1,2,3-triazolo-[4,5-b]pyridin-1-yl-methylene]-N-methylmethanaminium hexafluorophosphate N-oxide |
| LG | = | leaving group |
| m | = | multiplet |
| M | = | molar |
| M% | = | mole percent |
| max | = | maximum |
| meq | = | milliequivalent |
| mg | = | milligram |
| mL | = | milliliter |
| mm | = | millimeter |
| mmol | = | millimol |
| q | = | quartet |
| s | = | singlet |
| t or tr | = | triplet |
| TBS | = | tributylsilyl |
| TFA | = | trifluoroacetic acid |
| THF | = | tetrahydrofuran |
| TLC | = | thin layer chromatography |
| p-TLC | = | preparative thin layer chromatography |
| µL | = | microliter |
| N | = | normality |
| MeOH | = | methanol |
| DCM | = | dichloromethane |
| HCl | = | hydrochloric acid |
| ACN | = | acetonitrile |
| MS | = | mass spectrometry |
| rt | = | room temperature |
| EtOAc | = | ethyl acetate |
| EtO | = | ethoxy |
| Ac | = | acetate |
| NMP | = | 1-methyl-2-pyrrolidinone |
| µL | = | microliter |
| J | = | coupling constant |
| NMR | = | Nuclear magnetic resonance |
| MHz | = | megahertz |
| Hz | = | hertz |
| m/z | = | mass to charge ratio |
| min | = | minutes |
| Boc | = | *tert*-butoxycarbonyl |
| CBZ | = | benzyloxycarbonyl |
| DCC | = | 1,3-dicyclohexylcarbodiimide |
| PyBop | = | benzotriazole-1-yl-oxy-trispyrrolidinophosphonium hexafluorophosphate |

### General Methods

### General Method I

Step 1: The appropriate fluoronitrobenzene (**1**) is dissolved in NMP and Cooled to 0 °C. To the solution hünig's base and homomorpholine hydrochloride salt is added. The reaction is stirred at the appropriate temperature for about 18 h. Water is added and stirred for about 1 h resulting in the formation of a solid. The solid is collected via filtration, then rinsed with water and dried under vacuum to afford the desired nitro compound (**2**).
Step 2: The appropriate nitrobenzene (**2**) is dissolved in THF and placed in Parr shaker with Raney Nickel at 50 psi with a supply of H₂ for about 16 h. The resulting solution is filtered and concentrated to afford the desired aniline compound (**3**).
Step 3: The appropriate aniline (**3**) is dissolved in *tert*-butanol. To the solution oxirane-2-carboxylic acid methyl ester and lithium triflate is added. The reaction is heated at about 70 °C for about 16 h. To the reaction water and dichloromethane is added. The phase is separated and aqueous layer is extracted with dichloromethane. The combined organic layer is washed with brine and then with water, dried over Na₂SO₄ and concentrated *in vacuo*. The resulting oil is re-dissolved in dichloromethane. To the solution CDI is added and the reaction is stirred at rmt for about 16 h. The reaction is quenched with the addition of 10% citric acid and dichloromethane. The phase is separated and aqueous layer is extracted with dichloromethane. The combined organic layer is washed with brine and then with water, dried over Na₂SO4 and concentrated *in vacuo*. Purification by silica gel chromatography afforded the desired methyl ester compound (**5**).
Step 4: The appropriate methyl ester (5) is placed in a round bottom flask and the appropriate amine as a solution in methanol is added. The reaction is stirred at rmt for 5 h and then concentrated to afford the desired final product of the present invention.

The invention may also provide novel intermediates and novel processes that are useful for preparing compounds of formula I.

### EXAMPLES

### Example 1 Preparation of 3-(4-[1,4]oxazepan-4-yl-phenyl)-2-oxo-oxazolidine-5-carboxylic acid amide

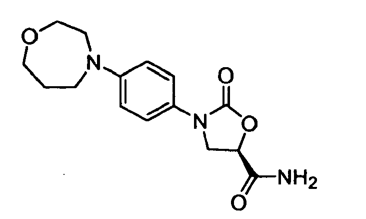

### Step 1: Preparation of 4-(4-nitro-phenyl)-[1,4]oxazepane

Following Step 1 of above general method, 2-fluoro nitrobenzene (500.0 mg, 3.54 mmol) in NMP (1.7 ml) with hünig's base (1.3 ml, 7.7 mmol) and homomorpholine hydrochloric salt (507.0 mg, 3.68 mmol) afforded the title compound as a solid.
¹H NMR (400 MHz, CDCl3): δ 2.05 (quint, 2 H), 3.70-3.74 (m, 6 H), 3.87 (t, 2 H), 6.67 (d, 2 H), 8.13 (d, 2 H). MS-APCI (m/z⁺): 223 (M+1).

### Step 2: Preparation of 4-[1,4]oxazepan-4-yl-phenylamine

Following Step 2 of above general method, 4-(4-nitro-phenyl)-[1,4]oxazepane (720.3 mg, 2.79 mmol) in THF (50 ml) with Raney Nickel (400.0 mg) afforded the title compound as oil.

¹H NMR (400 MHz, CDCl3): δ 2.03 (quint, 2 H), 3.50-3.53 (m, 4 H), 3.70 (t, 2 H), 3.82 (dd, 2 H), 6.62-6.67 (m, 4 H). MS-APCI (m/z⁺): 193 (M+1).

### Step 3: Preparation of 3-(4-[1,4]oxazepan-4-yl-phenyl)-2-oxo-oxazolidine-5-carboxylic acid methyl ester

Following Step 3 of above general method, 4-[1,4]oxazepan-4-yl-phenylamine (620.0 mg, 3.22 mmol) is reacted with oxirane-2-carboxylic acid methyl ester (311 l, 3.55 mmol) and lithium triflate (580.0 mg, 3.50 mmol) in *tert*-butanol (8.3 ml) and later with CDI (784.0 mg, 4.84 mmol) in dichloromethane (3 ml) to afford the title compound as a solid.

¹H NMR (400 MHz, CDCl3): δ 2.01 (quint, 2 H), 3.57-3.62 (m, 4 H), 3.66 (t, 2 H), 3.81 (t, 2 H), 3.86 (s, 3 H), 4.07 (dd, 1 H), 4.23 (t, 1 H), 5.03 (dt, 1H), 6.69 (d, 2 H), 7.28 (d, 2 H). MS-APCI (m/z⁺): 321 (M+1).

### Step 4: Preparation of 3-(4-[1,4]oxazepan-4-yl-phenyl)-2-oxo-oxazolidine-5-carboxylic acid amide:

Following Step 4 of above general method, 3-(4-[1,4]oxazepan-4-yl-phenyl)-2-oxo-oxazolidine-5-carboxylic acid methyl ester (72.4 mg, 0.23 mmol) is reacted with ammonia in methanol (2M, 1.1 ml, 2.3 mmol) to afford the title compound as a solid.

¹H NMR (400 MHz, DMSO-*d₆*): δ 1.82 (quint, 2 H), 3.47-3.50 (m, 6 H), 3.64 (t, 2 H), 3.85 (dd, 1 H), 4.14 (t, 1 H), 4.90 (dd, 1 H), 6.69 (d, 2 H), 7.25 (d, 2 H), 7.51 (s, 1 H), 7.74 (s, 1 H). MS-APCI (m/z⁺): 306 (M+2).

### Example 2 Preparation of 3-(4-[1,4]oxazepan-4-yl-phenyl)-2-oxo-oxazolidine-5-carboxylic acid methylamide

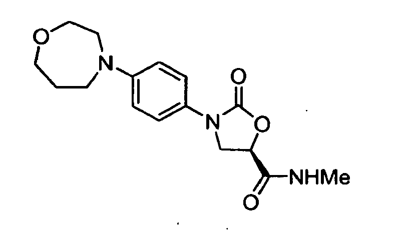

Following Step 4 of above general method, 3-(4-[1,4]oxazepan-4-yl-phenyl)-2-oxo-oxazolidine-5-carboxylic acid methyl ester (70.2 mg, 0.22 mmol) is reacted with methyl amine in methanol (2M, 1.1 ml, 2.2 mmol) to afford the title compound as a solid.¹H NMR (400 MHz, DMSO-*d₆*): δ 1.88 (quint, 2 H), 2.66 (d, 3 H), 3.53-3.57 (m, 6 H), 3.70 (t, 2 H), 3.92 (dd, 1 H), 4.20 (t, 1 H), 5.00 (dd, I H), 6.75 (d, 2 H), 7.31 (d, 2 H), 8.33 (q, 1 H). MS-APCI (m/z⁺): 320 (M+1).

### Example 3 Preparation of 3-(3-Fluoro-4-[1,4]oxazepan-4-yl-phenyl)-2-oxo-oxazolidine-5- carboxylic acid amide

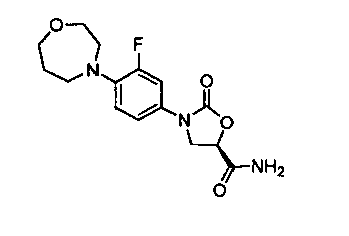

### Step 1: Preparation of 4-(2-fluoro-4-nitrophenyl)-[1.4]oxazepane

Following Step 1 of above general method, 3,4-difluoronitrobenzene (6.60 g, 41.49 mmol, 4.59 mL) in NMP (40 mL) is cooled to at about -20 °C (ice/salt water bath). Added Hunig's base (11.53 g, 89.19 mmol, 15.54 mL) followed by addition of homomorpholine HCl (5.99 g, 43.60 mmol) portion wise over approx. 30 min. After the addition is completed, the reaction solution is stirred for about 45 min. at 0 °C and the reaction is diluted with H2O (175 mL) to provide a yellow precipitate. The solid is collected by filtration; the solids are washed with H₂O (3x) and dried under house high vacuum on buchner funnel over night to afford the title compound.

¹H NMR (400 MHz, CHLOROFORM-D) δ ppm 2.00-2.06 (m, 2 H) 3.62-3.68 (m, 4 H) 3.74-3.77 (m, 2 H) 3.3.82-3.85 (m, 2 H) 6.74 (t, *J*=9.1 Hz, 1 H) 7.83-7.92 (m, 2 H).

### Step 2: Preparation of 3-fluoro-4-[1.4]oxazepan-4-yl-phenylamine

Following Step 2 of above general method, 4-(2-Fluoro-4-nitrophenyl)-[1.4]oxazepane (9.15 g, 38.10 mmol) is dissolved in tetrahydrofuran (200 mL) in a Parr shaker and placed in a H₂ atmosphere at 50 psi in the presence of Raney Ni for 15 hrs. The solution is then filtered and the solvent removed in vacuo to afford the title compound.

MS-APCI (*m*/*z*+): 211.

### Step 3: Preparation of 3-(3-fluoro-4-[1,4]oxazepan-4-yl-phenyl)-2-oxo-oxazolidine-5-carboxylic acid methyl ester

Following Step 3 of above general method, 3-fluoro-4-[1.4]oxazepan-4-yl-phenylamine (1.00 g, 4.76 mmol) is dissolved in t-butanol (15mL), methyl glycidate (0.483 g, 5.71 mmol, 0.500 mL) is added, heated to approx 40 °C in oil bath then added lithium triflate (0.890 g, 5.71 mmol) then raised temperature to 70 °C and stirred under N₂ at 70 °C overnight. Heat is then removed and H₂O added, extracted (2x) with dichloromethane, organics combined, washed with brine, dried over sodium sulfate, filtered, conc. The isolated residue is then dissolved in dichloromethane (40 mL), 1-hydroxybenzotriazole (0.354 g, 2.60 mmol) added and stirred under N2 at room temperature for 10 minutes. Carbonyldiimidazole (0.0.50 g, 3.08 mmol) is added and the reaction mixture heated at 35 ° in an oil bath overnight. Heating is then removed and additional dichloromethane added, washed organic phase with saturated ammonium chloride solution and then with brine, dried over sodium sulfate, filtered and solvent removed in vacuo. The isolated residue is subjected to silica gel flash chromatography, eluting with 20% MeOH in dichloromethane/dichloromethane gradient (0-10% of 20% MeOH in dichloromethane over 1 hr) to afford the title compound.

¹H NMR (400 MHz, CHLOROFORM-D) δ ppm 2.00-2.03 (m, 2 H) 3.38-3.41 (m, 4 H) 3.78-3.83 (m, 7 H) 4.04 (dd, *J*=9.2, 5.5 Hz, 1 H) 4.20 (t, *J*=9.4 Hz, 1 H) 5.01 (dd, *J*=9.6, 5.3 Hz, 1 H) 6.87 (br s, 1 H) 7.00-7.03 (m, 1 H) 7.30-7.33 (m, 1 H).

### Step 4: Preparation of 3-(3-fuoro-4-[1,4]oxazepan-4-yl-phenyl)-2-oxo-oxazolidine-5-carboxylic acid amide

Following Step 4 of above general method, 3-(3-Fluoro-4-[1,4]oxazepan-4-yl-phenyl)-2-oxo-oxazolidine-5-carboxylic acid methyl ester (0.50 g, 1.48 mmol) is dissolved in 7 mL of MeOH. Cooled in an ice bath and 2N NH₃/MeOH (4.43mL, 8.87 mmol) is added, ice bath removed and stirred for 2 1/2 hours at room temperature after which the solvent is removed in vacuo. MeOH added and cooled in ice bath. Resulting solids are filtered off and washed with cold MeOH, then with diethyl ether to afford the title compound.

¹H NMR (400 MHz, CHLOROFORM-D) δ ppm 2.00-2.03 (m, 2 H) 3.38-3.40 (m, 4 H) 3.78-3.83 (m, 4 H) 4.14 (dd, *J*=9.4, 5.9 Hz, 1 H) 4.21 (t, *J*=9.6 Hz, 1 H) 4.93 (dd, *J*=9.8, 5.9 Hz, 1 H) 5.72 (s, 1 H) 6.59 (s, 1 H) 6.88 (br s, 1 H) 6.98-7.01 (m, 1 H) 7.33 (dd, *J*=14.8, 2.5 Hz, 1 H)

### Example 4 Preparation of 3-(3-fluoro-4-[1,4]oxazepan-4-yl-phenyl)-2-oxo-oxazolidine-5-carboxylic acid methylamide

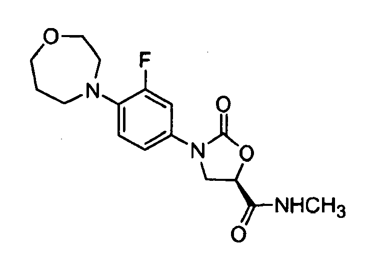

Following Step 4 of above general method, 3-(3-fluoro-4-[1,4]oxazepan-4-yl-phenyl)-2-oxo-oxazolidine-5-carboxylic acid methyl ester (0.40 g, 1.07 mmol) (0.32 g, 0.95 mmol) is dissolved in 4 mL of MeOH and cooled in an ice bath and 2N NH₃/MeOH (2.84 mL, 5.67 mmol) is added, ice bath removed and stirred for about 2 1/2 hours at room temperature after which the solvent is removed in vacuo. MeOH added and cooled in ice bath. Resulting solids are filtered off and washed with cold MeOH, then with diethyl ether to afford the title compound.

¹H NMR (400 MHz, CHLOROFORM-D) δ ppm 2.00-2.03 (m, 2 H) 2.86 (d, *J*=5.1 Hz, 3 H) 3.38-3.40 (m, 4 H) 3.78-3.83 (m, 4 H) 4.14 (dd, *J*=9.4, 5.7 Hz, 1 H) 4.20 (t, *J*=9.6 Hz, 1 H) 4.91 (dd, *J*=9.8, 5.5 Hz, 1 H) 6.71 (m, 1 H) 6.88 (br s, 1 H) 6.97-7.00 (m, 1 H) 7.33 (dd, *J*=14.8, 2.3 Hz, 1 H).

### Example 5 Preparation of 3-(3,5-difluoro-4-[1,4]oxazepan-4-yl-phenyl)-2-oxo-oxazolidine-5-carboxylic acid amide

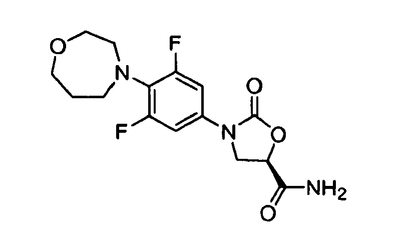

### Step 1: Preparation of 4-(2,6-difluoro-4-nitro-phenyl)-[1,4]oxazepane

Following Step 1 of above general method, 3,4,5-trifluoro nitrobenzene (5.03 g, 28.42 mmol) in MeCN (15 ml) with hünig's base (10.3 ml, 58.97 mmol) and homomorpholine hydrochloric salt (4.06 g, 29.5 mmol) afforded the title compound as a solid

¹H NMR (400 MHz, CDCl3): δ 2.02 (quint, 2 H), 3.53-3.58 (m, 4 H), 3.80-3.85 (m, 4 H), 7.73 (ddd, 2 H). MS-APCI (m/z⁺): 259 (M+1).

### Step 2: Preparation of 3,5-difluoro-4-[1,4]oxazepan-4-yl-phenylamine

Following Step 2 of above general method, 4-(2,6-difluoro-4-nitro-phenyl)-[1,4]oxazepane (624.3 mg, 2.42 mmol) in THF (50 ml) with Raney Nickel (300.0 mg) afforded the title compound as oil.

¹H NMR (400 MHz, CDCl3): δ 1.97 (quint, 2 H), 3.23-3.27 (m, 4 H), 3.78 (dd, 2 H), 3.91 (t, 2 H), 6.16-6.21 (m, 2 H). MS-APCI (m/z⁺): 229 (M+1).

### Step 3: Preparation of 3-(3,5-difluoro-4-[1,4]oxazepan-4-yl-phenyl)-2-oxo-oxazolidine-5-carboxylic acid methyl ester

Following Step 3 of above general method, 3,5-difluoro-4-[1,4]oxazepan-4-yl-phenylamine (2.0 g, 8.76 mmol) is reacted with oxirane-2-carboxylic acid methyl ester (843 µl, 9.64 mmol) and lithium triflate (1.6 g, 9.6 mmol) in *tert*-butanol (27 ml) and later with CDI (1.78 g, 11.0 mmol) in dichloromethane (44 ml) to afford the title compound as a solid.

¹H NMR (400 MHz, CDCl3): δ 2.00 (quint, 2 H), 3.32-3.37 (m, 4 H), 3.79-3.81 (m, 2 H), 3.87 (s, 3 H), 3.90 (t, 2 H), 4.07 (dd, 1 H), 4.21 (t, 1 H), 5.06 (dd, 1 H), 7.06-7.11 (m, 2 H). MS-APCI (m/z⁺): 357 (M+1).

### Step 4: Preparation of 3-(3,5-Difluoro-4-[1,4]oxazepan-4-yl-phenyl)-2-oxo-oxazolidine-5-carboxylic acid amide

Following Step 4 of above general method, 3-(3,5-difluoro-4-[1,4]oxazepan-4-yl-phenyl)-2-oxo-oxazolidine-5-carboxylic acid methyl ester (6) (151.4 mg, 0.42 mmol) is reacted with ammonia in methanol (2M, 2.1 ml, 4.2 mmol) to afford the title compound as a solid.

¹H NMR (400 MHz, DMSO-*d₆*): δ 2.00 (quint, 2 H), 3.30-3.35 (m, 4 H), 3.79 (dd, 2 H), 3.91 (t, 2 H), 4.07 (dd, 1 H), 4.30 (t, 1 H), 5.07 (dd, 1 H), 7.22-7.28 (m, 2 H). MS-APCI (m/z⁺): 342 (M+1).

### Example 6 Preparation of 3-(3,5-difluoro-4-[1,4]oxazepan-4-yl-phenyl)-2-oxo-oxazolidine-5-carboxylic acid methylamide

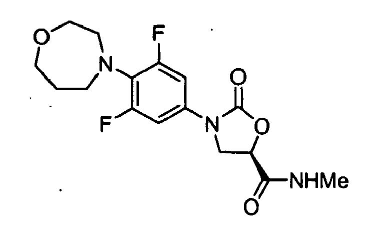

### Step 1 Preparation of 3-(3,5-difluoro-4-[1,4]oxazepan-4-yl-phenyl)-2-oxo-oxazolidine-5-carboxylic acid methylamide

Following Step 4of above general method, 3-(3,5-difluoro-4-[1,4]oxazepan-4-yl-phenyl)-2-oxo-oxazolidine-5-carboxylic acid methyl ester (131.7 mg, 0.37 mmol) is reacted with methylamine in methanol (2M, 1.8 ml, 3.7 mmol) to afford the title compound as a solid.

¹H NMR (400 MHz, (DMSO-*d₆*): δ 2.00 (quint, 2 H), 2.91 (d, 3 H), 3.33-3.37 (m, 4 H), 3.80 (dd, 2 H), 3.91 (dd, 2 H), 4.14-4.24 (m, 2 H), 4.97 (dd, 1 H), 6.59 (q, 1 H), 7.06-7.12 (m, 2 H). MS-APCI (m/z⁺): 359 (M+1).

### Example 7 Preparation of 2-oxo-3-(2,3,5-trifluoro-4-[1,4]oxazepan-4-yl-phenyl)oxazolidine-5-carboxylic acid amide

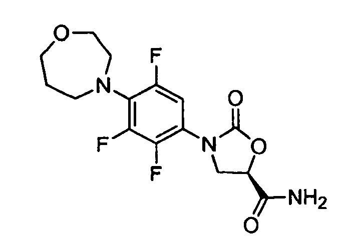

### Step 1: Preparation of 4-(2,3,6-trifluoro-4-nitrophenyl)-[1.4]oxazepane

Following Step 1 of above general method, tetrafluoronitrobenzene (6.75 g, 34.60 mmol, 8.29 mL) in NMP (33 mL) is cooled to at about -20 °C (ice/salt water bath). Added Hunig's base (9.62 g, 74.49 mmol, 12.96 mL) followed by addition of homomorpholine HCl (5.00 g, 36.30 mmol) portion wise over approx. 30 min. After the addition is completed, the reaction solution is stirred for 45 min. at 0 °C and the reaction is diluted with H₂O (175 mL) to provide a yellow precipitate. The solid is collected by filtration; the solids are washed with H₂O (3x) and dried under house high vacuum on buchner funnel over night to afford the title compound.

¹H NMR (400 MHz, CHLOROFORM-D) δ ppm 2.01-2.04 (m, 2 H) 3.58-3.63 (m, 4 H) 3.80-3.84 (m, 4 H) 7.60-7.65 (m, 1 H).

### Step 2: Preparation of 2,3,5-trifluoro-4-[1.4]oxazepan-4-yl-phenylamine

Following Step 2 of above general method, 4-(2,3,6-Trifluoro-4-nitrophenyl)-[1.4]oxazepane (10.42g, 37.72 mmol) is dissolved in tetrahydrofuran (200 mL) in a Parr shaker and place in a H₂ atmosphere at 50 psi in the presence of Raney Ni for 16 hrs. The solution is then filtered and the solvent removed in vacuo. The isolated residue is subjected to silica gel flash chromatography, eluting with MeOH/dichloromethane gradient (0-5% MeOH over 1 hour and 20 minutes) to afford the title compound.

MS-APCI (*m*/*z*+): 247.

### Step 3: Preparation of 2-oxo-3-(2,3,5-trifluoro-4-[1,4]oxazepan-4-yl-phenyl)oxazolidine-5-carboxylic acid methyl ester

Following Step 3 of above general method, 2,3,5-tTrifluoro-4-[1.4]oxazepan-4-yl-phenylamine (1.00 g, 4.06 mmol) is dissolved in t-butanol (15mL), methyl glycidate (0.456 g, 4.47 mmol, 0.39 mL).was added, heated to approx 40 °C in oil bath then added lithium triflate (0.697 g, 4.47 mmol) then raised temp. to 70 °C and stirred under N₂ at 70 °C overnight. An additional .40 eq of lithium triflate and methyl glycidate added and reaction mixture again stirred at 70 °C overnight. Heat removed, allowed to cool. H₂O added, extracted (2x) with dichloromethane, organics combined, washed with brine, dried over Sodium sulfate, filtered, conc. The isolated residue is then dissolved in dichloromethane (30 mL), 1-hydroxybenzotriazole (0.574 g, 4.25 mmol) added and stirred under N2 at room temperature for 10 minutes. Carbonyldiimidazole (0.788 g, 4.86 mmol) is added and the reaction mixture heated at 35 ° in an oil bath overnight. Heat removed, allowed to cool, diluted with additional dichloromethane, washed organic phase with saturated NH₄Cl and then with brine, dried over Sodium sulfate, filtered, conc. The isolated residue is subjected to silica gel flash chromatography, eluting with 20% MeOH in dichloromethane/dichloromethane gradient (0-10% of 20% MeOH in dichloromethane over 1 hr) to afford the title compound.

MS-APCI (*m*/*z*+): 375

### Step 4: Preparation of 2-oxo-3-(2,3,5-trifluoro-4-[1,4]oxazepan-4-yl-phenyl)oxazolidine-5-carboxylic acid amide

Following Step 4 of above general method, 2-Oxo-3-(2,3,5-trifluoro-4-[1,4]oxazepan-4-yl-phenyl)-oxazolidine-5-carboxylic acid methyl ester (0.40 g, 1.07 mmol) is dissolved in 7 mL of MeOH and cooled in an ice bath and 2N NH₃/MeOH (3.2 1 mL, 6.41 mmol) is added, ice bath removed and stirred for about 2 1/2 hours at room temperature after which the solvent is removed in vacuo. The isolated residue is subjected to silica gel flash chromatography, eluting with MeOH/dichloromethane gradient (0-10% MeOH over 1 hour and 10 minutes) to afford the title compound.

¹H NMR (400 MHz, CHLOROFORM-D) δ ppm 1.95-2.00 (m, 2 H) 3.37-3.42 (m, 4 H) 3.76-3.78 (m, 2 H) 3.84-3.87 (m, 2 H) 4.14 (dd, *J*=9.4, 5.9 Hz, 1 H) 4.24 (t, *J*=9.5 Hz, 1 H) 4.98 (dd, *J*=9.6, 5.9 Hz, 1 H) 5.07 (br s, 1 H) 6.55 (br s, 1 H) 6.96 (ddd, *J=*12.3, 6.8, 2.3 Hz, 1 H).

### Example 8 Preparation of 2-oxo-3-(2,3,5-trifluoro-4-[1,4]oxazepan-4-yl-phenyl)oxazolidine-5-carboxylic acid methylamide

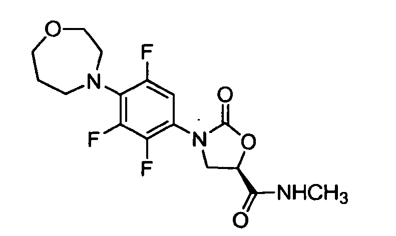

Following Step 1 of above general method, 2-oxo-3-(2,3,5-trifluoro-4-[1,4]oxazepan-4-yl-phenyl)-oxazolidine-5-carboxylic acid methyl ester (0.40 g, 1.07 mmol) is dissolved in 10 mL of MeOH and then cooled in an ice bath and 2N CH₃NH₂/MeOH (4.00mL, 8.00 mmol) is added, ice bath removed and stirred for 2 1/2 hours at room temperature after which the solvent is removed in vacuo. The isolated residue is subjected to silica gel flash chromatography, eluting with MeOH/dichloromethane gradient (0-10% MeOH over 1 hour and 10 minutes) to afford the title compound.

¹H NMR (400 MHz, CHLOROFORM-D) δ ppm 1.94-2.00 (m, *J*=5.9, 5.9, 5.9, 5.9 Hz, 2 H) 2.90 (d, *J*=4.9 Hz, 3 H) 3.38 (dd, *J*=9.1, 5.2 Hz, 3 H) 3.38 (s, 1 H) 3.75-3.78 (m, 2 H) 3.84-3.86 (m, 2 H) 4.12 (dd, *J*=9.2, 5.9 Hz, 1 H) 4.24 (t, *J*=9.1 Hz, 1 H) 4.96 (dd, *J*=9.7, 5.8 Hz, 1 H) 6.58 (br d, 1H), 6.960 (ddd, *J*=12.3, 7.0, 2.3 Hz, 1 H).

## Claims

1. A compound of formula I 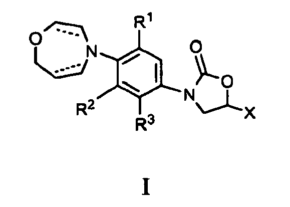 or a pharmaceutically acceptable salt thereof wherein:
X is -C(=O)NHR⁴;
R¹, R², R³ are independently H or F;
R⁴ is H, C₁₋₆ alkyl, or OC₁₋₆alkyl;
each dotted line "..." is independently an optional single bond; and at each occurrence, C₁₋₆alkyl is optionally substituted by 1-3 halo, OH, OC₁₋₄alkyl, CN, N₃, O(C=O)C₁₋₄ alkyl, NH₂, NHC(=O)C₁₋₄ alkyl, or C(=O)C₁₋₄ alkyl.

2. A compound of claim 1 which is a compound of formula Ia

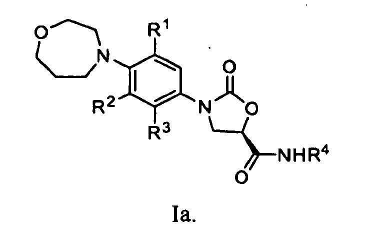

3. A compound of claim 2 wherein R⁴ is H, -CH₃, or -CH₂CH₃.

4. A compound of claim 2 wherein R⁴ is -OCH₃, or -OCH₂CH₃.

5. A compound of claim 1 which is
(1) 3-(4-[1,4]oxazepan-4-yl-phenyl)-2-oxo-oxazolidine-5-carboxylic acid amide,
(2) 3-(4-[1,4]oxazepan-4-yl-phenyl)-2-oxo-oxazolidine-5-carboxylic acid methylamide,
(3) 3-(3-Fluoro-4-[1,4]oxazepan-4-yl-phenyl)-2-oxo-oxazolidine-5-carboxylic acid amide,
(4) 3-(3-fluoro-4-[,4]oxazepan-4-yl-phenyl)-2-oxo-oxazolidine-5-carboxylic acid methylamide,
(5) 3-(3,5-difluoro-4-[1,4]oxazepan-4-yl-phenyl)-2-oxo-oxazolidine-5-carboxylic acid amide,
(6) 3-(3,5-difluoro-4-[1,4]oxazepan-4-yl-phenyl)-2-oxo-oxazolidine-5-carboxylic acid methylamide,
(7) 2-oxo-3-(2,3,5-trifluoro-4-[1,4]oxazepan-4-yl-phenyl)oxazolidine-5-carboxylic acid amide, or
(8) 2-oxo-3-(2,3,5-trifluoro-4-[1,4]oxazepan-4-yl-phenyl)oxazolidine-5-carboxylic acid methylamide.

6. A pharmaceutical composition comprising a compound as claimed in any one of claims I to 5 or a pharmaceutically acceptable salt thereof, and a pharmaceutically acceptable carrier.

7. A compound as claimed in any one of claims 1 to 5 for use in therapy.

8. A compound as claimed in any one of claims 1 to 5 for treating bacterial infections.

9. Use of a compound as claimed in any one of claims 1 to 5 for the manufacture of a medicament for treating bacteria infections.

10. The compound of claim 8 or the use of claim 9 wherein the compound is administered orally, parenterally, topically, rectally, or intranasally.

11. The compound of claim 8 or the use of claim 9 wherein said compound is administered in an amount of from about 0.1 to about 100 mg/kg of body weight/day.

12. The compound of claim 8 or the use of claim 9 wherein said compound is administered in an amount of from about 1 to about 50 mg/kg of body weight/day.

13. The compound of claim 8 or the use of claim 9 wherein the bacteria infection is selected from ear infections, eye infections, respiratory tract infections, skin and skin structure infections, bacterial endocarditis, osteomyelitis, endocarditis or diabetic foot.

14. The compound of claim 8 or the use of claim 9 wherein the bacteria infection is caused by gram-positive bacteria, gram negative bacteria, anaerobic organisms, and acid-fast organisms.

15. The compound of claim 8 or the use of claim 9 wherein the bacteria infection is caused by bacteria comprising staphylococci, streptococci, Enterococci, Haemophilus, Moraxella, bacteroides, clostridia, Mycobacteria, or Chlamydia.

16. The compound or the use of claim 13 wherein staphylococci is *S. aureus and S. epidermidis*; wherein streptococci is *S. pneumoniae* or *S. pyogenes*; wherein Enterococci is *E. faecalis*; wherein Haemophilus is *H. influenzae*; wherein Moraxella is *M. catarrhalis*; and wherein Mycobacteria is *M. tuberculosis*; or *Mycobacterium avium*.

17. The compound of claim 8 or the use of claim 9 wherein the bacteria infection is caused by multi-drug resistant S. aureus.

## Patentansprüche

1. Verbindung der Formel I 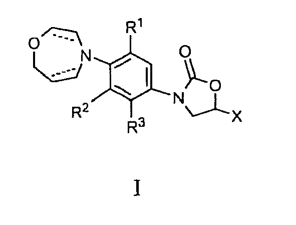 oder ein pharmazeutisch verträgliches Salz davon, worin:
X -C(=O)NHR⁴ ist;
R¹, R², R³ unabhängig H oder F sind;
R⁴ H, C₁₋₆-Alkyl oder OC₁₋₆-Alkyl ist;
jede punktierte Linie "..." unabhängig eine optionale Einfachbindung ist und
bei jedem Vorkommen C₁₋₆-Alkyl gegebenenfalls mit 1-3 Halogen, OH, OC₁₋₄-Alkyl, CN, N₃, O(C=O)C₁₋₄-Alkyl, NH₂, NHC(=O)C₁₋₄-Alkyl oder C(=O)C₁₋₄-Alkyl substituiert ist.

2. Verbindung gemäß Anspruch 1, die eine Verbindung der Formel Ia 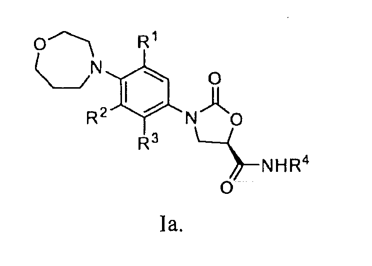 ist.

3. Verbindung gemäß Anspruch 2, wobei R⁴ H, -CH₃ oder -CH₂CH₃ ist.

4. Verbindung gemäß Anspruch 2, wobei R⁴ -OCH₃ oder -OCH₂CH₃ ist.

5. Verbindung gemäß Anspruch 1, welche
(1) 3-(4-[1,4]Oxazepan-4-yl-phenyl)-2-oxo-oxazolidin-5-carbonsäureamid,
(2) 3-(4-[1,4]Oxazepan-4-yl-phenyl)-2-oxo-oxazolidin-5-carbonsäure-methylamid,
(3) 3-(3-Fluor-4-[1,4]oxazepan-4-yl-phenyl)-2-oxo-oxazolidin-5-carbonsäureamid,
(4) 3-(3-Fluor-4-[1,4]oxazepan-4-yl-phenyl)-2-oxo-oxazolidin-5-carbonsäuremethylamid,
(5) 3-(3,5-Difluor-4-[1,4]oxazepan-4-yl-phenyl)-2-oxo-oxazolidin-5-carbonsäureamid,
(6) 3-(3,5-Difluor-4-[1,4]oxazepan-4-yl-phenyl)-2-oxo-oxazolidin-5-carbonsäuremethylamid,
(7) 2-Oxo-3-(2,3,5-trifluor-4-[1,4]oxazepan-4-yl-phenyl)oxazolidin-5-carbonsäureamid oder
(8) 2-Oxo-3-(2,3,5-trifluor-4-[1,4]oxazepan-4-yl-phenyl)oxazolidin-5-carbonsäuremethylamid
ist.

6. Pharmazeutische Zusammensetzung, umfassend eine Verbindung, wie sie in einem der Ansprüche 1 bis 5 beansprucht ist, oder ein pharmazeutisch verträgliches Salz davon und einen pharmazeutisch verträglichen Träger.

7. Verbindung, wie sie in einem der Ansprüche 1 bis 5 beansprucht ist, zur Verwendung in einer Therapie.

8. Verbindung, wie sie in einem der Ansprüche 1 bis 5 beansprucht ist, zur Behandlung bakterieller Infektionen.

9. Verwendung einer Verbindung, wie sie in einem der Ansprüche 1 bis 5 beansprucht ist, für die Herstellung eines Medikaments zur Behandlung von Bakterieninfektionen.

10. Verbindung gemäß Anspruch 8 oder Verwendung gemäß Anspruch 9, wobei die Verbindung oral, parenteral, topisch, rektal oder intranasal verabreicht wird.

11. Verbindung gemäß Anspruch 8 oder Verwendung gemäß Anspruch 9, wobei die Verbindung in einer Menge von etwa 0,1 bis etwa 100 mg/kg Körpergewicht/Tag verabreicht wird.

12. Verbindung gemäß Anspruch 8 oder Verwendung gemäß Anspruch 9, wobei die Verbindung in einer Menge von etwa 1 bis etwa 50 mg/kg Körpergewicht/Tag verabreicht wird.

13. Verbindung gemäß Anspruch 8 oder Verwendung gemäß Anspruch 9, wobei die Bakterieninfektion ausgewählt ist aus Infektionen des Ohrs, Infektionen des Auges, Infektionen der Atemwege, Infektionen der Haut und der Hautstruktur, bakterieller Endokarditis, Osteomyelitis, Endokarditis oder Diabetikerfuß.

14. Verbindung gemäß Anspruch 8 oder Verwendung gemäß Anspruch 9, wobei die Bakterieninfektion durch Gram-positive Bakterien, Gram-negative Bakterien, anaerobe Organismen und säurebeständige Organismen verursacht wird.

15. Verbindung gemäß Anspruch 8 oder Verwendung gemäß Anspruch 9, wobei die Bakterieninfektion durch Bakterien verursacht wird, die Staphylococci, Streptococci, Enterococci, Haemophilus, Moraxella, Bacteroides, Clostridia, Mycobacteria oder Chlamydia umfassen.

16. Verbindung oder Verwendung gemäß Anspruch 13, wobei Staphylococci *S. aureus* und *S. epidermidis* sind; wobei Streptococci *S. pneumoniae* oder *S. pyogenes* sind; wobei Enterococci *E. faecalis* sind; wobei Haemophilus *H. influenzae* ist; wobei Moraxella *M. catarrhalis* ist und wobei Mycobacteria *M. tuberculosis* oder *Mycobacterium avium* sind.

17. Verbindung gemäß Anspruch 8 oder Verwendung gemäß Anspruch 9, wobei die Bakterieninfektion durch mehrfach resistenten S. aureus verursacht wird.

## Revendications

1. Composé de formule I 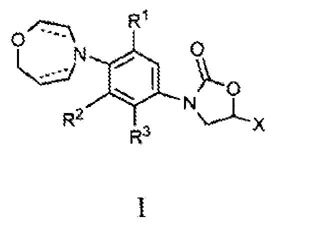 ou sel pharmaceutiquement acceptable de celui-ci, dans lequel :
X représente -C(=O)NHR⁴ ;
R¹, R², R³ représentent indépendamment H ou F ;
R⁴ représentant H, alkyle en C₁₋₆ ou O(alkyle en C₁₋₆ ) ;
chaque ligne en pointillés "..." représente indépendamment une liaison simple facultative ; et
à chaque apparition, alkyle en C₁₋₆ est facultativement substitué par de 1 à 3 groupe(s) halogéno, OH, O(alkyle en C₁₋₄), CN, N₃, O(C=O)(alkyle en C₁₋₄), NH₂, NHC(=O)(alkyle en C₁₋₄) ou C(=O)(alkyle en C₁₋₄).

2. Composé selon la revendication 1, qui est un composé de formule la

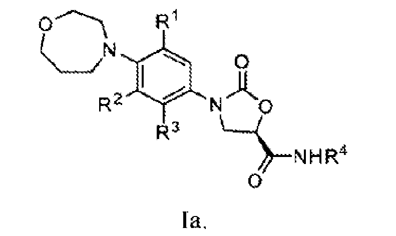

3. Composé selon la revendication 2, dans lequel R⁴ représente H, -CH₃ ou -CH₂CH₃.

4. Composé selon la revendication 2, dans lequel R⁴ représente -OCH₃ ou -OCH₂CH₃.

5. Composé selon la revendication 1, qui est :
(1) l'amide de l'acide 3-(4-[1,4]oxazépan-4-yl-phényl)-2-oxo-oxazolidine-5-carboxylique,
(2) le méthylamide de l'acide 3-(4-[1,4]oxazépan-4-yl-phényl)-2-oxo-oxazolidine-5-carboxylique,
(3) l'amide de l'acide 3-(3-fluoro-4-[1,4]oxazépan-4-yl-phényl)-2-oxo-oxazolidine-5-carboxylique,
(4) le méthylamide de l'acide 3-(3-fluoro-4-[1,4]oxazépan-4-yl-phényl)-2-oxo-oxazolidine-5-carboxylique,
(5) l'amide de l'acide 3-(3,5-difluoro-4-[1,4]oxazépan-4-yl-phényl)-2-oxo-oxazolidine-5-carboxylique,
(6) le méthylamide de l'acide 3-(3,5-difluoro-4-[1,4]oxazépan-4-yl-phényl)-2-oxo-oxazolidine-5-carboxylique,
(7) l'amide de l'acide 2-oxo-3-(2,3,5-trifluoro-4-[1,4]oxazépan-4-yl-phényl)oxazolidine-5-carboxylique ou
(8) le méthylamide de l'acide 2-oxo-3-(2,3,5-trifluoro-4-[1,4]oxazépan-4-yl-phényl)oxazolidine-5-carboxylique.

6. Composition pharmaceutique comprenant un composé tel que revendiqué dans l'une quelconque des revendications 1 à 5, ou un sel pharmaceutiquement acceptable de celui-ci, et un support pharmaceutiquement acceptable.

7. Composé tel que revendiqué dans l'une quelconque des revendications 1 à 5, pour une utilisation en thérapie.

8. Composé tel que revendiqué dans l'une quelconque des revendications 1 à 5, pour traiter des infections bactériennes.

9. Utilisation d'un composé tel que revendiqué dans l'une quelconque des revendications 1 à 5, pour la fabrication d'un médicament pour traiter des infections bactériennes.

10. Composé selon la revendication 8 ou utilisation selon la revendication 9, le composé étant administré par voie orale, parentérale, topique, rectale ou intranasale.

11. Composé selon la revendication 8 ou utilisation selon la revendication 9, ledit composé étant administré en une quantité allant d'environ 0,1 à environ 100 mg/kg de poids corporel/jour.

12. Composé selon la revendication 8 ou utilisation selon la revendication 9, ledit composé étant administré en une quantité allant d'environ 1 à environ 50 mg/kg de poids corporel/jour.

13. Composé selon la revendication 8 ou utilisation selon la revendication 9, l'infection bactérienne étant sélectionnée parmi les infections de l'oreille, les infections de l'oeil, les infections des voies respiratoires, les infections cutanées ou sous-cutanées, l'endocardite bactérienne, l'ostéomyélite, l'endocardite et le pied diabétique.

14. Composé selon la revendication 8 ou utilisation selon la revendication 9, l'infection bactérienne étant causée par des bactéries Gram-positives, des bactéries Gram-négatives, des organismes anaérobies et des organismes acido-résistants.

15. Composé selon la revendication 8 ou utilisation selon la revendication 9, l'infection bactérienne étant causée par des bactéries comprenant les staphylocoques, les streptocoques, les entérocoques, les Haemophilus, les Moraxella, les Bacteroides, les Clostridia, les mycobactéries ou les Chlamydia.

16. Composé ou utilisation selon la revendication 13, dans lequel/laquelle les staphylocoques sont *S. aureus* et *S. epidermidis* ; les streptocoques sont *S. pneumoniae* et *S. pyogenes* ; les entérocoques sont *E. faecalis* ; les Haemophilus sont *H. influenzae* ; les Moraxella sont *M. catarrhalis ;* et les mycobactéries sont *M. tuberculosis* ou *Mycobacterium avium.*

17. Composé selon la revendication 8 ou utilisation selon la revendication 9, l'infection bactérienne étant causée par *S. aureus* multi-résistant aux médicaments.
